# EUROPEAN PATENT SPECIFICATION

(11) **EP 1 386 905 B1**
(45) Date of publication and mention of the grant of the patent: **26.09.2012**
(21) Application number: 02724742.8
(22) Date of filing: 09.05.2002
(51) Int. Cl.: C07C 41/46, A61K 9/127, A23L 1/30, A23K 1/16

(54) **STABLE SOLUTION OF REDUCED COENZYME Q**
REDUZIERTES COENZYM Q ENTHALTENDE, STABILE LÖSUNG
SOLUTION STABLE DE COENZYME Q REDUITE

(30) Priority: 09.05.2001 JP 2001138340
(43) Date of publication of application: 04.02.2004
(73) Proprietor: KANEKA CORPORATION, Osaka-shi, Osaka 530-8288 (JP)
(72) Inventor: FUJII, Kenji, Kobe-shi, Hyogo 651-1202 (JP); KAWABE, Taizo, Himeji-shi, Hyogo 672-8044 (JP); HOSOE, Kazunori, Takasago-shi, Hyogo 676-0025 (JP); HIDAKA, Takayoshi, Kobe-shi, Hyogo 655-0006 (JP)
(74) Representative: HOFFMANN EITLE
(86) International application number: PCT/JP2002/004516
(87) International publication number: WO 2002/090304

(56) References cited:
- EP-A1- 0 522 433
- EP-A2- 0 069 399
- WO-A-01/52822
- WO-A-02/04025
- WO-A-02/17879
- WO-A1-94/18948
- WO-A1-98/07417
- GB-A- 2 184 355
- JP-A- 53 056 315
- JP-A- 59 161 314
- TAKAYUKI TAKAHASHI ET AL.: "Reduction of ubiquinone in membrane lipids by rat liver cytosol and its involvement in the cellular defence system against lipid peroxidation" BIOCHEMICAL JOURNAL., vol. 309, no. 3, 1995, pages 883-890, XP008044034 GBTHE BIOCHEMICAL SOCIETY, LONDON.
- TAKEO KISHI ET AL.: "Ubiquinone redox cycle as a cellular antioxidant defense system" BIOFACTORS., vol. 10, no. 2-3, 1999, pages 131-138, XP008043930 GBOXFORD UNIVERSITY PRESS, OXFORD.
- YORIHIRO YAMAMOTO ET AL.: "Antioxidant activity of ubiquinol in solution and phosphatidylcholine liposome" JOURNAL OF NUTRITIONAL SCIENCE AND VITAMINOLOGY., vol. 36, no. 5, 1990, pages 505-511, XP008044046 JPUNIVERSITY OF TOKYO PRESS, TOKYO

## Description

### Technical Field

The present invention relates to a solution containing reduced coenzyme Q as a component, and particularly to a solution which can stably maintain reduced coenzyme Q against oxidation.

### Background Art

Coenzyme Q is an essential component which is distributed in a wide variety of living organisms ranging from bacteria to mammals, and is known as a component of the electron transport system of cellular mitochondria in living organisms. It is also known that coenzyme Q undergoes oxidation/reduction cycles in the mitochondria and functions as an electron carrier in the electron transport system, and reduced coenzyme Q has an antioxidative effect. Human coenzyme Q is mainly composed of coenzyme Q₁₀, having 10 repeat structures in its side chain, and about 40% to 90% of coenzyme Q present in living organisms is generally in its a reduced form. Examples of physiological functions of coenzyme Q include the activation of energy production through activating mitochondrial function, activation of cardiopulmonary function, stabilization of cellular membranes, production of cells through an antioxidative effects, and the like.

Coenzyme Q is used in various applications, for example, oxidized coenzyme Q₁₀ is used in the treatment of a congestive heart failure due to its effects on the heart. In addition to therapeutic usage, coenzyme Q is orally used as a nutritional supplement or a nutritional adjuvant like vitamins. However, coenzyme Q is insoluble in water because of its hydrophobicity, and is thus only actually used as an oral agent and a skin agent.

In recent years, the exacerbation of various diseases due to an increase in oxidative stress in the blood has been reported. Typical examples of such diseases include arterial sclerosis, diabetic complications, and the like. These diseases are caused or exacerbated by lipid degeneration due to various types of oxidative stress in the blood. In order to decrease the influence of the oxidative stress, it is effective to administer an antioxidative agent to activate antioxidative ability. Vitamin E, a typical lipid-soluble antioxidative compound thought to be effective for suppressing lipid peroxidation, is widely used as an antioxidant for preventing diseases. It has recently been reported that the coexistence of reduced coenzyme Q₁₀ is important for vitamin E to exhibit its the antioxidative activity sufficiently (Bowry et al., 1993, J. American Chemical Society, 115, 6029-6044), and the importance of coenzyme Q as well as vitamin E has been recognized as a lipid-soluble antioxidant.

Since reduced coenzyme Q has a strong antioxidative effect by itself, the antioxidative activity in the blood can be effectively increased by supplying a sufficient amount of solubilized reduced coenzyme Q. By increasing the antioxidative activity in the blood, beneficial effects on many diseases that ate exacerbated due to reactive oxygen species, such as the prevention of angiopathy in ischemic recirculation and restenosis of arterial sclerosis, the prevention of angiopathy after cerebral infarction, the prevention of arterial sclerosis, the prevention of diabetic complications, etc., can be expected. Furthermore, using a new delivery form of reduced coenzyme Q, reduced coenzyme Q can be transferred into the body by a drip, and is thus useful for patients with serious conditions or patients with cerebropathy who cannot orally intake reduced coenzyme Q. In this way, it is expected that solubilization of reduced coenzyme Q will have many benefits for maintaining good health.

There have previously been many studies on methods for solubilizing oxidized coenzyme Q₁₀ (ubidecarenone or ubiquinone). Various methods for solubilizing oxidized coenzyme Q₁₀, such as coating with a liposome, suspending with a surfactant or fat or oil, etc. have been reported (Japanese Unexamined Patent Application Publication Nos. 5-186340 and 7-69874 and PCT Japanese Translation Patent Publication No. 2000-510841). However, none of these methods have been put into practical use. One reason for this is that in order for oxidized coenzyme Q₁₀ to exhibit antioxidative activity, the oxidized coenzyme Q₁₀ must be converted into its reduced form by a reducing enzyme, but the amount of reduced coenzyme Q₁₀ in the blood cannot be increased by injection administration because of the absence of the reducing enzyme in the blood, and therefore a decrease in blood oxidative stresses cannot be expected by an increase in the antioxidative activity. On the other hand, reduced coenzyme Q₁₀ itself has antioxidative activity and is thus a promising substance for the above-described diseases. However, reduced coenzyme Q₁₀ has not been put into practical use because of its tendency to undergo oxidation and instability. The production of liposome-coated reduced coenzyme Q₁₀ for studying an oxidation-reduction enzyme has been reported (Kishi et al., 1999, BioFactors, 10, 131-138). However, this report only discloses methods for preparing liposomes immediately before use, and a solubilizing method for stably maintaining reduced coenzyme Q is not known.

### Summary of the Invention

An object of the present invention is to provide an aqueous solution capable of stably maintaining reduced coenzyme Q against oxidation.

As a result of studies to overcome the above-described problem, the inventors found a solution having a composition suitable for stably maintaining reduced coenzyme Q against oxidation, leading to the realization of the present invention. That solution is defined in claims 1 to 11.

The solution of the present invention can be used in medical products, food products, food compositions, drinks, fertilizers, cosmetics, animal feedstuff, or an antioxidant. Examples of a medical product include an injection, a fluid replacement, a liquid drug, eye drops, nose drops, ear drops, an oral agent, a skin agent, a scalp agent, and a stock solution. Besides humans, these medical products can be used for animals such as dogs, cats, rabbits, rats, mice, cows, horses, pigs, and the like.

The present invention also provides a method for stabilizing reduced coenzyme Q comprising coating the reduced coenzyme Q with a liposome prepared from a purified soybean keithin.

### Detailed Disclosure of Invention

The present invention will be described in detail below. Coenzyme Q is represented by formulae (1) and (2): 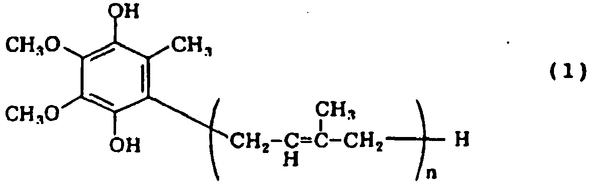 (wherein n represents an integer of 1 to 12); 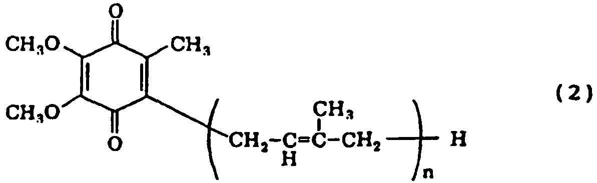 (wherein n represents an integer of 1 to 12). Reduced coenzyme Q is represented by formula (1), and oxidized coenzyme Q is represented by formula (2).

Coenzyme Q used in the present invention contains reduced coenzyme Q represented by formula (1), in which the lower limit of the amount of reduced coenzyme Q in the total amount of coenzyme Q is preferably 20% by weight, and more preferably 40% by weight, and the upper limit of the amount may be 100% by weight or less, preferably less than 100% by weight, and more preferably 98% by weight or less. With respect to the ratio of reduced coenzyme Q in coenzyme Q, it was found that the effect of increasing the amount of blood coenzyme Q₁₀ by administering an oral composition using reduced coenzyme Q₁₀ is increased with a reduced coenzyme Q₁₀ content of 20% by weight or more, and is significantly increased with a content of 40% by weight or more (Japanese Unexamined Patent Application Publication No. 10-109933).

The method for obtaining reduced coenzyme Q is not limited, and an example of a usable method comprises obtaining coenzyme Q by a conventional known process such as synthesis, fermentation, extraction from a natural source, or the like, and then concentrating a reduced coenzyme Q fraction from a chromatography eluate can be used. In this case, if required, a general reducing agent such as sodium borohydride, sodium dithionite (sodium hydrosulfite), or the like may be added to the coenzyme Q, for reducing oxidized coenzyme Q contained in the coenzyme Q to form reduced coenzyme Q by a conventional process, and then the obtained reduced coenzyme Q may be concentrated by chromatography. Reduced coenzyme Q can also be obtained by the reaction of the reducing agent with existing coenzyme Q of high purity.

As the coenzyme Q used in the present invention, as shown in formulae (1) and (2), a coenzyme having 1 to 12 (n in each formula) repeat units in its side chain can be used. Particularly, a coenzyme having 10 side chain repeat units, i.e., coenzyme Q₁₀, can be preferably used.

The method for obtaining the solution of the present invention is not limited, and for example, the solution can be obtained by coating the coenzyme Q containing the reduced coenzyme Q with a liposome using an appropriate base to solubilize the coenzyme Q. The solution can also be obtained by solubilization or emulsification with an appropriate surfactant.

As a lipid used as the base for the liposome, a phospholipid is used. In the present invention, that phospholipid is purified soybean keithin.

The content of phosphatidyl choline of the purified soybean lecithin is preferably 0.01% by weight or more, and more preferably 0.1% by weight or more.

With respect to the composition ratio of the coenzyme Q to the phospholipid, 1 mole or more of phospholipid may be present relative to 1 mole of coenzyme Q, but 10 moles or more of phospholipid are preferable.

Also, sterol may be added to the base. The upper limit of the amount of sterol added is preferably 1/5, and more preferably 1/10 of the weight of phospholipid. As the sterol, cholesterol is most preferably used, but another sterol may be used.

Although the content of the coenzyme Q in the solution of the present invention depends upon what the solution is to be used for, the lower limit is preferably 0.0001% by weight, and the upper limit is preferably 50% by weight. More preferably, the lower limit is 0.001% by weight, and the upper limit is 30% by weight, and most preferably, the lower limit is 0.01% by weight, and the upper limit is 15% by weight.

Liposome coating can be performed by a process known to persons skilled in the art. For example, a solvent of a mixture of phospholipid and reduced coenzyme Q dissolved in the solvent such as chloroform, ethanol, or the like is evaporated to dryness, and then the residue is dispersed in an appropriate buffer by an ultrasonic treatment or the like. The above operation is preferably performed at a low temperature (for example, 4°C) in an inert gas atmosphere of nitrogen or the like, for suppressing oxidation of the reduced coenzyme Q and the lipid.

Next, solubilization or emulsification with a surfactant will be described.

As the surfactant, a commonly used product may be used. Examples of the surfactant include anionic surfactants such as a carboxylate anionic surfactant, a sulfonate anionic surfactant, a sulfate anionic surfactant, a phosphate anionic surfactant, and the like; cationic surfactants such as an amine salt cationic surfactant, a quaternary ammonium salt cationic surfactant, and the like; ampholytic surfactants such as an aminocarboxylate ampholytic surfactant, a carboxybetaine ampholytic surfactant, a sulfate ampholytic surfactant, a sulfonic acid ampholytic surfactant, and the like; nonionic surfactants such as an ether nonionic surfactant, an ether ester nonionic surfactant, an ester nonionic surfactant, a block polymer nonionic surfactant, a nitrogen-containing nonionic surfactant, and the like; other surfactants such as a natural surfactant, a surfactant derived from a protein hydrolysate, a polymeric surfactant, a surfactant containing titanium and silicon, a fluorocarbon surfactant, and the like.

As the surfactant, a nonionic surfactant is preferred, and a poly-solvate surfactant such as Tween 80 or the like, or polyoxyethylene hardened castor oil such as HCO 60 or the like is more preferred.

The concentration of the surfactant in the solution of the present invention is 0.01 to 1%, by weight from the viewpoint of antioxidation of the reduced coenzyme Q, and most preferably 0.1% by weight or less. The content of the coenzyme Q is the same as that for liposome coating.

The solution prepared as described above may further contain pharmaceutically allowable raw materials for drug formulations, which are appropriately added by a conventional method. The raw materials for drug formulations which can be added are not limited, and for example, an emulsifier, a tensing agent, a buffer, a solubilizing agent, a flavoring agent, an antiseptic agent, a stabilizer, an antioxidant, and the like may be added.

The method for preserving the solution composition of the present invention is not limited, and a conventional method such as cold storage, anaerobic storage using an airtight container, light-shielding storage, or the like may be used.

When the solution of the present invention prepared as described above is stored at a low temperature or room temperature, the reduced coenzyme Q can be stably maintained against oxidation. The phrase "can be stably maintained" means that the residual amount of the reduced coenzyme Q is 80% or more of the concentration at the start of storage. The storage period during which this percentage is maintained is preferably 1 week or more, more preferably 1 month or more, and most preferably 1 year or more.

The solution containing the reduced coenzyme Q according to the present invention can be widely used for medical treatment, cosmetics, foods, gardening, dairy products, and the like. Example applications of the solution include an injection, an infusion solution, a fluid drug, eye drops, a fluid drug for internal application, a lotion agent, a hair tonic, a cosmetic emulsion, a spray liquid, an aerosol, a drink, a liquid fertilizer, a storage solution, and the like. In medical applications, the solution can also be used as a storage solution for organ transplant. Furthermore, the solution may be used for animals, fish and seafood, and used as animal feedstuff. The solution can also be used as an antioxidative solution for storing fresh food such as meat, fish, and the like.

### Brief Description of the Drawings

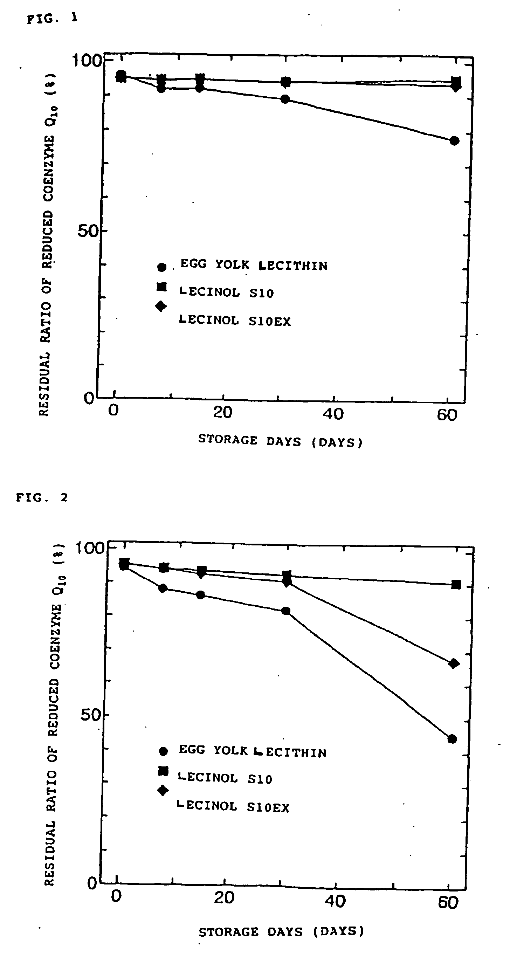

Fig. 1 is a graph showing the oxidation stability at 4°C of reduced coenzyme Q₁₀ in a liposome prepared using each of three types of lecithin in which the residual amount of reduced coenzyme Q₁₀ to the content at the time of preparation is shown by % on the ordinate, the number of storage days is shown on the abscissa, and each value is an average with n = 2.
Fig. 2 is a graph showing the oxidation stability at 23°C of reduced coenzyme Q₁₀ in a liposome prepared using each of three types of lecithin in which the residual amount of reduced coenzyme Q₁₀ to the content at the time of preparation is shown by % on the ordinate, the number of storage days is shown on the abscissa, and each value is an average with n = 2.

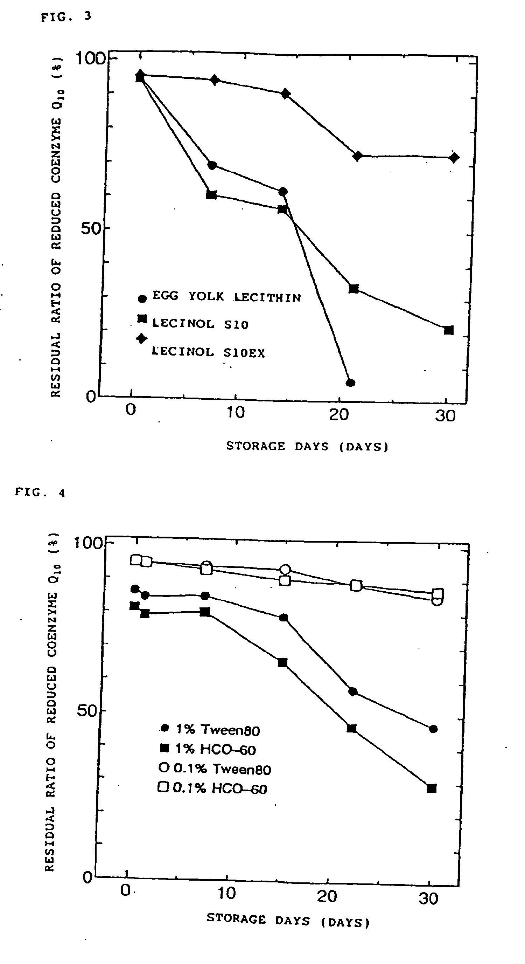

Fig. 3 is a graph showing the oxidation stability at 40°C of reduced coenzyme Q₁₀ in a liposome prepared using each of three types of lecithin in which the residual amount of reduced coenzyme Q₁₀ to the content at the time of preparation is shown by % on the ordinate, the number of storage days is shown on the abscissa, and each value is an average with n = 2.
Fig. 4 is a graph showing the influence of surfactant concentration on the oxidation stability at 23°C of reduced coenzyme Q₁₀ prepared with each of two types of surfactant in which the residual amount of reduced coenzyme Q₁₀ to the content at the time of preparation is shown on the ordinate, the number of storage days is shown on the abscissa, and each value is an average with n = 2.

### Best Mode for Carrying Out the Invention

Although the present invention will be described in further detail below with reference to examples and preparation examples, the present invention is not limited to these examples and preparation examples.

### (EXAMPLE 1) Influence of lecithin on oxidation stability of reduced coenzyme Q₁₀ in a liposome containing reduced coenzyme Q₁₀

As the lecithin, egg yolk lecithin (produced by Wako Pure Chemical Industries, Ltd.) and purified soybean lecithin Lecinol S10 and Lecinol S10EX (produced by Nihon Chemicals Co., Ltd.) were used. Lecinol S10 contains 25 to 30% by weight of phosphatidyl choline, and Lecinol S10EX contains 95% by weight or more of phosphatidyl choline. A liposome containing reduced coenzyme Q₁₀ was prepared using the lecithin as described below. Namely, a lecithin-chloroform solution (3.2 mg/ml) was added to an ethanol solution (1 mg/ml) of coenzyme Q₁₀ (oxidized type:reduced type = 5:95 (ratio by weight)), and dissolved. After the solvent was completely removed by an evaporator under reduced pressure, HEPES buffer (50 mM, pH 7.4) was added to the residue, and a lipid film was dispersed by shaking to prepare a milky-white suspension. The suspension was subjected to ultrasonic treatment at 4°C for 30 minutes in a nitrogen stream to prepare a liposome, and macromolecules were removed by centrifugation. In order to prevent oxidation of the reduced coenzyme Q₁₀ during the operation, the operation was carried out as rapidly as possible.

The oxidation stability of the reduced coenzyme Q₁₀ in the liposome was evaluated by high performance liquid chromatography (HPLC).

The amount of reduced coenzyme Q₁₀ in the liposome containing 0.05% of reduced coenzyme Q₁₀ immediately after preparation was about 90% by weight using egg yolk lecithin, and about 95% by weight using purified soybean lecithin (Lecinol S10 and Lecinol S10EX). It was thus found that the reduced coenzyme Q₁₀ is slightly oxidized during preparation of the liposome using the egg yolk lecithin, while the reduced coenzyme Q₁₀ is little oxidized during preparation of the liposome using the purified soybean lecithin.

Also, the influence of storage temperature on oxidation of the reduced coenzyme Q₁₀ was examined by storing the liposome in air. The results are shown in Figs. 1 to 3, in which each value is an average with n = 2.

In cold storage (4°C) for 30 days, 90% or more of the reduced coenzyme Q₁₀ was maintained in the liposome prepared using each of the three types of lecithin. After storage for 60 days, the reduced coenzyme Q₁₀ was little oxidized in the liposome prepared using Lecinol S10 or Lecinol S10EX, while about 25% of the reduced coenzyme Q₁₀ was oxidized in the liposome prepared using the egg yolk lecithin (Fig. 1).

After storage at 23°C for 30 days, 90% or more of the reduced coenzyme Q₁₀ in the liposome prepared using either Lecinol S10 or Lecinol S10EX was maintained in a reduced state, and 80% or more of the reduced coenzyme Q₁₀ in the liposome prepared using the egg yolk lecithin was maintained in a reduced state. However, after storage for 60 days, 90% or more of the reduced coenzyme Q₁₀ remained in the liposome with Lecinol S10, while with Lecinol S10EX and the egg yolk lecithin, about 35% and about 55%, respectively, of the reduced coenzyme Q₁₀ were oxidized (Fig. 2).

After heating at 40°C with the egg yolk lecithin, the reduced coenzyme Q₁₀ was mostly oxidized. While with Lecinol S10, the reduced coenzyme Q₁₀ was decreased by about 50% after storage for 2 weeks, and the amount of reduced coenzyme Q₁₀ remaining after 30 days was about 20%. With the liposome using Lecinol S10EX, about 70% of the reduced coenzyme Q₁₀ remained as a reduced type after 30 days (Fig. 3).

As a result, it was found that purified soybean lecithin Lecinol is suitable as a liposome base for the reduced coenzyme Q₁₀, compared with egg yolk lecithin. Particularly, purified soybean lecithin Lecinol S10EX having a high content of phosphatidyl choline is stable at 40°C. However, Lecinol S10 exhibits higher stability at 25°C, and thus a stable storage period can be obtained by appropriately selecting the purified soybean lecithin according to the storage conditions for the product used. In applications requiring long-term storage, purified soybean lecithin, particularly lecithin having a high content of phosphatidyl choline, is excellent as the base. (EXAMPLE 2) Stability of solubilizing solution of reduced coenzyme Q₁₀ using a surfactant

A solution of reduced coenzyme Q₁₀ using a surfactant was prepared by the method described below, and the oxidation stability under storage was evaluated. As the surfactant, Polysolvate 80 (Tween 80) or polyoxyethylene hardened castor oil (HCO 60) was used, and an aqueous solution of 1% by weight or 0.1% by weight of the surfactant was prepared. Then, the reduced coenzyme Q₁₀ was added to the aqueous solution so that the final concentration of the solution was 0.05% by weight. Fig. 4 shows the influence of the surfactant concentration on the oxidation stability of the reduced coenzyme Q₁₀ during air storage of the solution at 23°C. In Fig. 4, each value is an average with n = 2. It was found that the oxidation stability of the reduced coenzyme Q₁₀ is strongly influenced by the surfactant concentration, and the solution prepared with a low surfactant concentration has high stability. The results of this test indicate that with a surfactant concentration of 0.1% by weight, the reduced coenzyme Q₁₀ can be stably stored at 23°C for about 1 month. While with a surfactant concentration of 1% by weight, the reduced coenzyme Q₁₀ can be stably stored for 1 week, and is rapidly oxidized thereafter. Therefore, when preparing a solution or emulsion with a surfactant, the concentration of the surfactant used is decreased to prepare a solution which can be stored for a longer period of time. A solution or emulsion with 1% by weight or more of a surfactant can be used under conditions in which the solution or emulsion is prepared before use, or the solution or emulsion is stored for a short period or stored in an airtight condition such as a completely anaerobic condition.

### (PREPARATION EXAMPLE 1) Injection

| | |
|---|---|
| Purified soybean lecithin | 0.3% by weight |
| Ethanol | 6.5% by weight |
| Macrogol 400 | 5.0% by weight |
| Sorbitol | 4.5% by weight |
| Reduced coenzyme Q₁₀ | 0.1% by weight |
| Distilled water for injection | 100.0% by weight |

### (PREPARATION EXAMPLE 2) Emulsion

| | |
|---|---|
| Tween 80 | 1.0% by weight |
| Glycerin | 12.5% by weight |
| Phosphatidyl choline | 1.2% by weight |
| Reduced coenzyme Q₁₀ | 0.1% by weight |
| Purified water | 100.0% by weight |

### (PREPARATION EXAMPLE 3) Face lotion

| | |
|---|---|
| Purified soybean lecithin | 0.2% by weight |
| Squalane | 0.1% by weight |
| Ethanol | 14.0% by weight |
| Glycerin | 4.0% by weight |
| Reduced coenzyme Q₁₀ | 0.1% by weight |
| Purified water | 100.0% by weight |

### Industrial Applicability

According to the present invention, reduced coenzyme Q having broad applicability as an antioxidant or nutraceutrical component can be supplied in the form of a liquid drug which permits application to a wide range of various fields.

## Claims

1. A solution of coenzyme Q containing reduced coenzyme Q represented by formula (1), wherein the coenzyme Q is coated with a liposome which is prepared from a purified soybean lecithin or solubilized or emulsified with a surfactant, wherein the concentration of the surfactant in the solution is 0.01 to less than 1% by weight, to stably maintain the reduced coenzyme Q against oxidation: 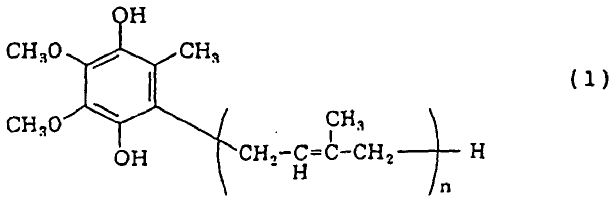 (wherein n represents an integer of 1 to 12).

2. A solution according to claim 1, wherein the content of the reduced coenzyme Q is 20% by weight or more of the total amount of the coenzyme Q.

3. A solution according to claim 1, wherein the content of the reduced coenzyme Q is 40% by weight or more of the total amount of the coenzyme Q.

4. A solution according to claim 1, wherein the concentration of the coenzyme Q is 0.0001 to 50% by weight.

5. A solution according to claim 1, wherein the coenzyme Q is coenzyme Q₁₀.

6. A solution according to any one of claims 1 to 5, further comprising sterol as a component of a liposome membrane.

7. A solution according to any one of claims 1 to 5, wherein the surfactant is at least one selected from the group consisting of a carboxylate anionic surfactant, a sulfonate anionic surfactant, a sulfate anionic surfactant, a phosphate anionic surfactant, an amine salt cationic surfactant, a quaternary ammonium salt cationic surfactant, an aminocarboxylate ampholytic surfactant, a carboxybetaine ampholytic surfactant, a sulfate ampholytic surfactant, a sulfonic acid ampholytic surfactant, an ether nonionic surfactant, an ether ester nonionic surfactant, an ester nonionic surfactant, a block polymer nonionic surfactant, a nitrogen-containing nonionic surfactant, a natural surfactant, a surfactant derived from a protein hydrolysate, a polymer surfactant, a surfactant containing titanium and silicon, and a fluorocarbon surfactant.

8. A solution according to claim 7, wherein the surfactant is polysolvate or polyoxyethylene hardened castor oil.

9. A solution according to any one of claims 1 to 8, wherein the solution is used as a medical product, a food product, a food composition, a drink, a fertilizer, a cosmetic, an animal feedstuff, or an antioxidant.

10. A solution according to any one of claims 1 to 8, wherein the solution is used as an injection, a fluid replacement, a liquid drug, eye drops, nose drops, ear drops, an oral agent, a skin agent, a scalp agent, or a stock solution.

11. A method for stabilizing reduced coenzyme Q comprising coating the reduced coenzyme Q with a liposome prepared from a purified soybean lecithin.

## Patentansprüche

1. Coenzym Q-Lösung, die reduziertes Coenzym Q, dargestellt durch die Formel (1), enthält, wobei das Coenzym Q mit einem Liposom, das aus gereinigtem Sojabohnenlecithin hergestellt wurde, beschichtet oder gelöst oder mit einem Tensid emulgiert ist, wobei die Konzentration des Tensids in der Lösung 0,01 bis weniger als 1 Gew.% beträgt, um das reduzierte Coenzym Q gegenüber Oxidation stabil zu halten: 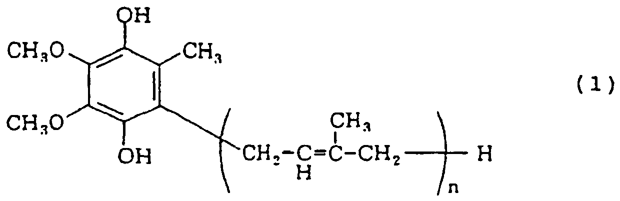 (wobei n eine ganze Zahl von 1 bis 12 darstellt).

2. Lösung gemäß Anspruch 1, wobei der Anteil des reduzierten Coenzyms Q 20 Gew.% oder mehr der Gesamtmenge des Coenzyms Q beträgt.

3. Lösung gemäß Anspruch 1, wobei der Anteil des reduzierten Coenzyms Q 40 Gew.% oder mehr der Gesamtmenge des Coenzyms Q beträgt.

4. Lösung gemäß Anspruch 1, wobei die Konzentration des Coenzyms Q 0,0001 bis 50 Gew.% beträgt.

5. Lösung gemäß Anspruch 1, wobei das Coenzym Q Coenzym Q₁₀ ist.

6. Lösung gemäß einem der Ansprüche 1 bis 5, die weiterhin Sterol als Bestandteil einer Liposomen-Membran umfasst.

7. Lösung gemäß einem der Ansprüche 1 bis 5, wobei das Tensid mindestens eines ist ausgewählt aus der Gruppe bestehend aus einem anionischen Carboxylattensid, einem anionischen Sulfonattensid, einem anionischen Sulfattensid, einem anionischen Phosphattensid, einem kationischen Aminsalztensid, einem kationischen quartären Ammoniumsalztensid, einem amphoteren Aminocarboxylattensid, einem amphoteren Carboxybetaintensid, einem amphoteren Sulfattensid, einem amphoteren Sulfonsäuretensid, einem nicht-ionischen Ethertensid, einem nicht-ionischen Etherestertensid, einem nicht-ionischen Estertensid, einem nicht-ionischen Blockpolymertensid, einem nicht-ionischen stickstoffhaltigen Tensid, einem natürlichen Tensid, einem Tensid, das von einem Proteinhydrolysat stammt, einem Polymertensid, einem Tensid, das Titan und Silizium enthält, und einem Fluorkohlenstofftensid.

8. Lösung gemäß Anspruch 7, wobei das Tensid Polysolvat - oder Polyoxyethylen-gehärtetes Rizinusöl ist.

9. Lösung gemäß einem der Ansprüche 1 bis 8, wobei die Lösung als Medizinprodukt, Lebensmittel, Lebensmittelzusammensetzung, Getränk, Düngemittel, Kosmetikprodukt, Tierfuttermittel oder Antioxidationsmittel verwendet wird.

10. Lösung gemäß einem der Ansprüche 1 bis 8, wobei die Lösung als Injektion, Flüssigkeitsersatz, flüssiges Arzneimittel, Augentropfen, Nasentropfen, Ohrentropfen, oraler Wirkstoff, Hautwirkstoff, Kopfhautwirkstoff oder Stammlösung verwendet wird.

11. Verfahren zur Stabilisierung von reduziertem Coenzym Q, das die Beschichtung von reduziertem Coenzym Q mit einem Liposom, das aus gereinigtem Sojabohnenlecithin hergestellt wurde, umfasst.

## Revendications

1. Solution de coenzyme Q contenant du coenzyme Q réduit représenté par la formule (1), dans laquelle le coenzyme Q est revêtu d'un liposome qui est préparé à partir d'une lécithine de soja purifiée ou solubilisé ou émulsifié avec un surfactant, où la concentration du surfactant dans la solution est de 0,01 à moins de 1 % en poids, pour maintenir de manière stable le coenzyme Q réduit contre l'oxydation: 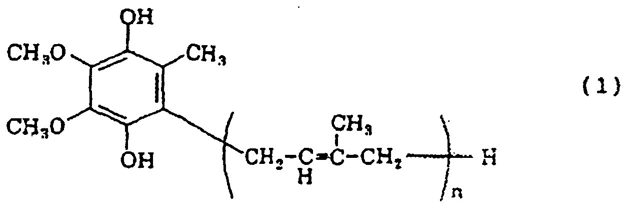 (où n représente un nombre entier de 1 à 12).

2. Solution selon la revendication 1, dans laquelle la teneur en coenzyme Q réduit est de 20 % en poids ou plus de la quantité totale du coenzyme Q.

3. Solution selon la revendication 1, dans laquelle la teneur en coenzyme Q réduit est de 40 % en poids ou plus de la quantité totale du coenzyme Q.

4. Solution selon la revendication 1, dans laquelle la concentration du coenzyme Q est de 0,0001 à 50 % en poids.

5. Solution selon la revendication 1, dans laquelle le coenzyme Q est le coenzyme Q₁₀.

6. Solution selon l'une quelconque des revendications 1 à 5, comprenant en outre du stérol en tant que composant d'une membrane liposomiale.

7. Solution selon l'une quelconque des revendications 1 à 5, dans laquelle le surfactant est au moins un surfactant choisi dans le groupe constitué d'un surfactant anionique de carboxylate, d'un surfactant anionique de sulfonate, d'un surfactant anionique de sulfate, d'un surfactant anionique de phosphate, d'un surfactant cationique d'un sel d'amine, d'un surfactant cationique d'un sel d'ammonium quaternaire, d'un surfactant ampholytique d'aminocarboxylate, d'un surfactant ampholytique de carboxybétaïne, d'un surfactant ampholytique de sulfate, d'un surfactant ampholytique d'acide sulfonique, d'un surfactant non ionique d'éther, d'un surfactant non ionique d'ester d'éther, d'un surfactant non ionique d'ester, d'un surfactant non ionique de polymère séquencé, d'un surfactant non ionique contenant de l'azote, d'un surfactant naturel, d'un surfactant dérivé d'un hydrolysat de protéines, d'un surfactant de polymère, d'un surfactant contenant du titane et du silicium et d'un surfactant de fluorocarbure.

8. Solution selon la revendication 7, dans laquelle le surfactant est du polysolvate ou de l'huile de ricin polyoxyéthylénée solidifiée.

9. Solution selon l'une quelconque des revendications 1 à 8, où la solution est utilisée en tant que produit médical, produit alimentaire, composition alimentaire, boisson, fertilisant, produit cosmétique, produit alimentaire pour animaux ou antioxydant.

10. Solution selon l'une quelconque des revendications 1 à 8, où la solution est utilisée sous la forme d'une injection, d'un remplacement de liquide, d'un médicament liquide, de gouttes pour les yeux, de gouttes pour le nez, de gouttes pour les oreilles, d'agent oral, d'agent cutané, d'agent pour le cuir chevelu ou de solution mère.

11. Procédé de stabilisation du coenzyme Q réduit comprenant le revêtement du coenzyme Q réduit d'un liposome préparé à partir de lécithine de soja purifiée.
